# NEUE EUROPÄISCHE PATENTSCHRIFT

(11) **EP 1 940 347 B2**
(45) Veröffentlichungstag und Bekanntmachung des Hinweises auf die Entscheidung über den Einspruch: **14.07.2021**
(45) Hinweis auf die Patenterteilung: 09.10.2013
(21) Anmeldenummer: 06791747.6
(22) Anmeldetag: 31.08.2006
(51) Int. Cl.: A61K 8/37, A61K 8/89, A61K 8/892, A61Q 5/06

(54) **FETTSÄUREESTER ZUR VERRINGERUNG DER KLEBRIGKEIT VON STYLINGMITTELN**
FATTY ACID ESTERS FOR REDUCING THE STICKINESS OF STYLING COMPOSITIONS
ESTER D'ACIDE GRAS DESTINE A DIMINUER LE CARACTERE COLLANT D'AGENTS COIFFANTS

(30) Priorität: 25.10.2005 DE 102005051333
(43) Veröffentlichungstag der Anmeldung: 09.07.2008
(73) Patentinhaber: Henkel AG & Co. KGaA, 40589 Düsseldorf (DE)
(72) Erfinder: KNAPPE, Thorsten, 22869 Schenefeld (DE); EMMERLING, Winfried, 25436 Tornesch (DE); BAYERSDÖRFER, Rolf, 22589 Hamburg (DE); BERGEMANN, Uwe, 22459 Hamburg (DE); SCHEFFLER, René, 25479 Ellerau (DE)
(74) Vertreter: LKGLOBAL Lorenz & Kopf PartG mbB Patentanwälte
(86) Internationale Anmeldenummer: PCT/EP2006/008504
(87) Internationale Veröffentlichungsnummer: WO 2007/048461

(56) Entgegenhaltungen:
- EP-A2- 0 705 595
- EP-A2- 0 705 595
- EP-A2- 0 928 608
- WO-A-2005/053624
- WO-A1-2004/030642
- JP-A- H1 179 953
- JP-A- H07 258 039
- US-A- 5 985 256
- US-A1- 2003 086 897
- US-B2- 6 365 144
- Lever Fabergé: "Styling Range", , 1 March 2005 (2005-03-01),
- Schwarzkopf & Henkel: "Hair Repair Fixing Spray", , 1 March 2001 (2001-03-01),
- Alberto Culver: "Brush Out Hair Spray Repackaging", , 1 February 2005 (2005-02-01),

## Beschreibung

Die vorliegende Erfindung betrifft die Verwendung bestimmter Fettsäureester zur Verringerung der Klebrigkeit von Stylingmitteln.

Stylingmittel zur Verformung keratinischer Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Stylingmittel müssen eine ganze Reihe unterschiedlicher Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Entscheidend ist natürlich zunächst eine hohe Festigerleistung, darüber hinaus aber auch Feuchtebeständigkeit, niedrige Klebrigkeit und ein ausgewogener Konditioniereffekt. Weiterhin soll ein Stylingmittel möglichst für alle Haartypen universell einsetzbar sein.

Gerade eine zu hohe Klebrigkeit wird vom Anwender eines Stylingmittels als sehr unangenehm empfunden. Es wurden daher eine Reihe spezieller festigender Polymere entwickelt, die sich neben einer guten Festigerleistung durch eine vergleichsweise niedrige Klebrigkeit auszeichnen (siehe z.B. WO 2005/053624 A1).

Diese Polymere sind aber in der Regel komplex aufgebaut und entsprechend aufwendig in der Herstellung und konnten sich daher nicht durchsetzen. Die überwiegende Zahl der heute im Markt befindlichen Stylingmittel basiert immer noch auf klassischen filmbildenden und/oder festigenden Polymeren, insbesondere Homo- und Copolymeren des Vinylpyrrolidons, etwa die Vinylpyrrolidon-Vinylacetat-Copolymer-Typen, oder dem amphoteren Copolymer aus Octylacrylamid, Acrylaten und/oder Methacrylaten und Butylaminoethylmethacrylat. Letzteres wird insbesondere zur Herstellung von Haarsprays, eingesetzt. Diese klassischen filmbildenden und/oder festigenden Polymere weisen jedoch eine hohe Klebrigkeit auf.

Aufgabe der vorliegenden Erfindung war es daher, die Klebrigkeit von Stylingmitteln, insbesondere solchen, die auf klassischen filmbildenden und/oder festigenden Polymeren basieren, herabzusetzen.

Es wurde nunmehr gefunden, dass dies durch Zusatz spezieller Fettsäureester zu Stylingmitteln erreicht werden kann.

Gegenstand der vorliegenden Erfindung ist daher die Verwendung mindestens eines Fettsäureesters der Formel (I) 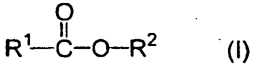 wobei
R¹ für den Kohlenstoffrest einer gesättigten oder ungesättigten, verzweigten oder unverzweigten Fettsäure, und
R² für gegebenenfalls verzweigtes C₁-C₂₀-Alkyl, gegebenenfalls verzweigtes C₁-C₂₀-Monohydroxyalkyl, gegebenenfalls verzweigtes C₂-C₂₀-Dihydroxyalkyl oder gegebenenfalls verzweigtes C₃-C₂₀-Trihydroxyalkyl steht,
zur Reduktion der Klebrigkeit in kosmetischen Mitteln zur temporären Verformung keratinischer Fasern,
wobei die kosmetischen Mittel zur temporären Verformung keratinischer Fasern als filmbildendes und/oder festigendes Polymer mindestens ein Homo- oder Copolymer des Vinylpyrrolidons oder ein amphoteres Copolymer aus Octylacrylamid, Acrylaten und/oder Methacrylaten und Butylaminoethylmethacrylat enthalten.

Unter keratinischen Fasern sind dabei erfindungsgemäß Pelze, Wolle, Federn und insbesondere menschliche Haare zu verstehen.

Der Einsatz der Fettsäureester der Formel (I) in kosmetischen Mitteln zur temporären Verformung keratinischer Fasern, d.h. in Stylingmitteln, verringert die Klebrigkeit der Haare nach Anwendung der Stylingmittel deutlich. Auf diese Kohlenstoffrest einer gesättigten oder ungesättigten, verzweigten oder unverzweigten C₆-C₃₀-Fettsäure steht.

Unter dem Kohlenstoffrest einer Festsäure wird dabei der Bestandteil der Fettsäure verstanden, der sich formal durch Abspaltung der Carbonsäuregruppe von der Fettsäure ergibt. So ist etwa der Kohlenstoffrest der Essigsäure eine Methylgruppe, der Kohlenstoffrest der Heptansäure eine Hexylgruppe, der Kohlenstoffrest der Tetradecansäure (Myristinsäure) eine Tridecanylgruppe oder der Kohlenstoffrest der Ölsäure eine Heptadecengruppe.

Besonders bevorzugt werden Fettsäureester der Formel (I) eingesetzt, bei denen R¹ für den Kohlenstoffrest einer gesättigten oder ungesättigten, verzweigten oder unverzweigten C₁₂-C₂₀-Fettsäure steht.

Ganz besonders bevorzugt werden Fettsäureester der Formel (I) eingesetzt, bei denen R¹ für den Kohlenstoffrest einer natürlich vorkommenden Fettsäure steht.

Der Alkoholteil der verwendeten Fettsäureester der Formel (I) leitet sich von C₁-C₂₀-Alkoholen mit bis zu 4 Hydroxygruppen ab.

Vorzugsweise werden Fettsäureester der Formel (I) eingesetzt, bei denen R² für gegebenenfalls verzweigtes C₁-C₁₀-Alkyl oder gegebenenfalls verzweigtes C₁-C₁₀-Monohydroxyalkyl steht. Besonders bevorzugt steht R² für gegebenenfalls verzweigtes C₁-C₄-Alkyl oder gegebenenfalls verzweigtes C₁-C₄-Monohydroxyalkyl, ganz besonders bevorzugt für Ethyl, n- Propyl, iso- Propyl, n- Butyl, iso- Butyl, tert.- Butyl oder eine Monohydroxybutylgruppe.

Erfindungsgemäß besonders bevorzugt ist die Verwendung eines Fettsäureesters der Formel (I) ausgewählt aus der Gruppe, die von Isopropylpalmitat, Isopropylmyristat, 1,2-Butylenglycololeat, 1,3-Butylenglycololeat, 1,4-Butylenglycololeat und 2,3- Butylenglycololeat gebildet wird. Ganz besonders bevorzugt ist der Einsatz von Isopropylmyristat.

Die Menge an Fettsäureester der Formel (I), die zum Einsatz kommt, ist so zu wählen, dass einerseits die gewünschte Verringerung der Klebrigkeit eines Stylingmittels erreicht, andererseits aber dessen primäre Eigenschaft, nämlich dem Haar Halt in der gewünschten Form zu verleihen, nicht negativ beeinflusst wird. In der Regel wird der oder die Fettsäureester der Formel (I) in einer Menge von 0,05 bis 2 Gew.-%, bezogen auf die Gesamtmenge an kosmetischem Mittel zur temporären Verformung keratinischer Fasern, eingesetzt. Bevorzugt ist der Einsatz in einer Menge von 0,1 bis 1 Gew.-%, besonders bevorzugt in einer Menge von 0,3 bis 1 Gew.-%.

Die Fettsäureester der Formel (I) werden daher zur Verringerung der Klebrigkeit von Stylingmitteln eingesetzt, die als filmbildendes und/oder festigendes Polymer mindestens ein Homo- oder Copolymer des Vinylpyrrolidons oder ein amphoteres Copolymer aus Octylacrylamid, Acrylaten und/oder Methacry- laten und Butylaminoethylmethacrylat enthalten.

Beispielhaft seien genannt Homopolymere des Vinylpyrrolidons, Copolymerisate aus Vinylpyrrolidon und Vinylacetat, z.B. Luviskol^{®} VA 37 oder PVP/VA Copolymer 60/40 W NP, Terpolymere aus Vinylpyrrolidon, Vinylacetat und Vinylpropionat, und das unter der Bezeichnung Amphomer^{®} erhältliche amphotere Octylacrylamid-Acrylat-Butylaminoethyl-Methacrylat- Copolymer.

Besonders gute Ergebnisse lassen sich erzielen, wenn der Fettsäureester der Formel (I) in Kombination mit einer Silikonverbindung eingesetzt wird. Die zusätzliche Verwendung einer Silikonverbindung ist daher bevorzugt.

Als Silikonverbindung eignen sich insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Geeignet sind etwa Dimethiconole (S1) . Diese können sowohl linear als auch verzweigt als auch cyclisch oder cyclisch und verzweigt sein. Lineare Dimethiconole können durch die folgende Strukturformel (S1-I) dargestellt werden:

(OHSiR¹₂)-O-(SiR²₂-O-)ₓ-(SiR¹₂OH) (S1-I)

Verzweigte Dimethiconole können durch die Strukturformel (S1-II) dargestellt werden:

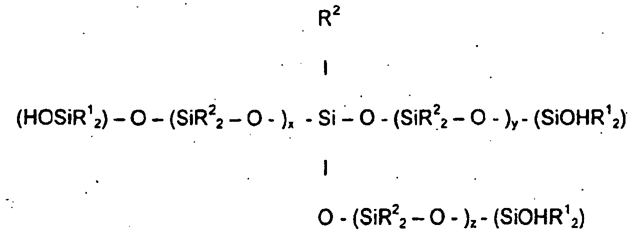

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, insbesondere bevorzugt ist R¹ und R² Methyl. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401 DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS (beide Guardian Laboratories), Nonychosine E, Nonychosine V (beide Exsymol), SanSurf Petrolatum-25, Satin Finish (beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Belsil DM 3112 VP, Wacker-Belsil DM 8005 VP, Wacker-Belsil DM 60081 VP (alle zuvor genannten Wacker-Chemie GmbH).

Weiterhin geeignet sind Dimethicone (S2). Diese können sowohl linear als auch verzweigt als auch cyclisch oder cyclisch und verzweigt sein. Lineare Dimethicone können durch die folgende Strukturformel (S2-I) dargestellt werden:

(SiR¹₃)-O-(SiR¹R²-O-)ₓ-(SiR¹₃) (S2-I)

Verzweigte Dimethicone können durch die Strukturformel (S2-II) dargestellt werden:

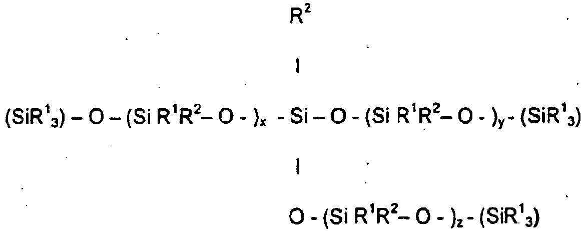

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und besonders bevorzugt ist R¹ und R² Methyl. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, besonders bevorzugte Viskositäten liegen zwischen 10000 und 3000000 cPs. Ganz besonders bevorzugt liegt die Viskosität im Bereich zwischen 50000 und 2000000 cPs.

Geeignete Silikonverbindungen sind überdies Dimethiconcopolyole (S3). Dimethiconcopolyole können durch die folgenden Strukturformeln dargestellt werden:

(SiR¹₃)-O-(SiR²₂-O-)ₓ-(SiR²PE-O-)y-(SiR¹₃) (S3-I).

PE-(SiR¹₂)-O-(SiR²₂-O-)ₓ-(SiR¹₂)-PE (S3-II)

Verzweigte Dimethiconcopolyole können durch die Strukturformel (S3-III) dargestellt werden: 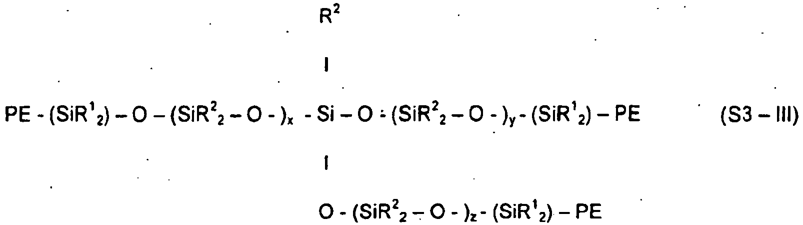 oder durch die Strukturformel (S3-IV):

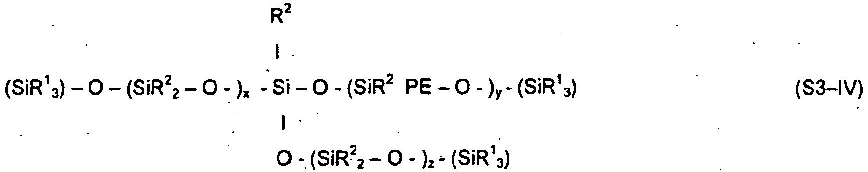

Die Reste R¹ und R² stehen unabhängig voneinander jeweils für Wasserstoff, einen Methylrest, einen C₂ bis C₃₀ linearen, gesättigten oder ungesättigten Kohlenwasserstoffrest, einen Phenylrest und/oder einen Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, insbesondere bevorzugt ist R¹ und R² Methyl.

PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Vsikositäten liegen zwischen 10000 und 3000000 cPs. Der bevorzugteste Bereich liegt zwischen 50000 und 2000000 cPs.

Vorzugsweise werden als Silikonverbindung die genannten Dimethiconcopolyole (S3) eingesetzt. Besonders bevorzugt ist die Verwendung von Dimethiconcopolyolen, deren Polyoxyalkylenreste sich von Ethylenoxid und/oder Propylenoxid ableiten. Insbesondere bevorzugt sind Dimethiconcopolyole, deren Polyoleinheiten sich sowohl von Ethylenoxid, als auch von Propylenoxid ableiten, wobei dabei wiederum die Dimethiconcopolyole bevorzugt sind; die im Durchschnitt mit der gleichen Anzahl an Ethylenoxid und Propylenoxid alkoxyliert sind. Entsprechende Dimethiconcopolyole sind kommerziell erhältlich und werden beispielsweise von der Firma Dow Corning unter der Bezeichnung Dow Corning^{®} 5330 Fluid (INCI-Bezeichnung: PEG/PPG-15/15 Dimethicone) oder Dow Corning^{®} 190 Surfactant (INCI-Bezeichnung: PEG/PPG-18/18 Dimethicone) vertrieben. Ganz besonders bevorzugt wird als Silikonverbindung Dow Corning^{®} 190 Surfactant (INCI-Bezeichnung: PEG/PPG-18/18 Dimethicone) eingesetzt.

Selbstverständlich umfasst die erfindungsgemäße Lehre auch, dass die Dimethiconole, Dimethicone und/oder Dimethiconcopolyole bereits als Emulsion vorliegen können. Dabei kann die entsprechende Emulsion der Dimethiconole, Dimethicone und/oder Dimethiconcopolyole sowohl nach der Herstellung der entsprechenden Dimethiconole, Dimethicone und/oder Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole, Dimethicone und/oder Dimethiconcopolyole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconole, Dimethicone und/oder Dimethiconcopolyole als Emulsion verwendet werden, dann beträgt die Tröpfchengröße der emulgierten Teilchen erfindungsgemäß 0,01 bis 10000 µm, bevorzugt 0,01 bis 100 µm, besonders bevorzugt 0,01 bis 20 µm und ganz besonders bevorzugt 0,01 bis 10 µm. Die Teilchengröße wird dabei nach der Methode der Lichtstreuung bestimmt.

Werden verzweigte Dimethiconole, Dimethicone und/oder Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Erfindung ist daher unter verzweigten Dimethiconolen, Dimethiconen und/oder Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole, Dimethicone und/oder Dimethiconcopolyole ganz besonders bevorzugt sein.

Geeignete Silikonverbindungen sind weiterhin aminofunktionelle Silikone (S4), insbesondere die Silikone, die unter der INCI-Bezeichnung Amodimethicone zusammengefasst sind. Darunter sind Silikone zu verstehen, welche mindestens eine, gegebenenfalls substituierte, Aminogruppe aufweisen.

Solche Silikone können z.B. durch die Formel (S4-I)

M(RₐQ_{b}SiO_{(4-a-b)/2)})ₓ(R_{c}SiO_{(4-c)/2)})_{y}M (S4-I)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel- R'Z ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silikon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH₂-, -CH₂CH₂OCH₂-, -OCH₂CH₂-, -OCH₂CH₂CH₂-, -CH₂CH(CH₃)C(O)OCH₂-, -(CH₂)₃C(O)OCH₂CH₂-, -C₆H₄C₆H₄-, -C₆H₄CH₂C₆H₄- ; und- (CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)₂NH₂, worin z für eine ganze Zahl von 1 bis 50 steht. Eine andere mögliche Formel für Z ist- NH(CH₂)_{z}NH(CH₂)_{zz}, worin sowohl z als auch zz unabhängig voneinander für eine ganze Zahl von 1 bis 50 stehen, wobei diese Struktur Diamino-Ringstrukturen umfasst, wie Piperazinyl. Z ist insbesondere bevorzugt ein-NHCH₂CH₂NH₂-Rest. Eine andere mögliche Formel für Z ist- N(CH₂)_{z}NX¹X² oder- NX¹X², worin X¹ und X² jeweils unabhängig voneinander ausgewählt ist aus Wasserstoff und einem Kohlenwasserstoffrest mit 1 bis etwa 6 Kohlenstoffatomen.

Ganz besonders bevorzugt steht Q für einen polaren, aminfunktionellen Rest der Formel-CH₂CH₂CH₂NHCH₂CH₂NH₂.

Das molare Verhältnis der RₐQ_{b} SiO_{(4-a-b)/2}-Einheiten zu den R_{c}SiO_{(4-c)/2}-Einheiten liegt im Bereich von etwa 1 : 2 bis 1 : 65, vorzugsweise von etwa 1 : 5 bis etwa 1 : 65 und besonders bevorzugt von etwa 1 : 15 bis etwa 1 : 20. Werden ein oder mehrere Silikone der obigen Formel eingesetzt, dann können die verschiedenen variablen Substituenten in der obigen Formel bei den verschiedenen Silikonkomponenten, die in der Silikonmischung vorhanden sind, verschieden sein.

Bevorzugte aminofunktionelle Silikone entsprechen der Formel (S4-II)

R^{'}ₐG₃₋ₐ-Si(OSiG₂)ₙ-(OSiG_{b}R'_{2-b)m}-O-SiG₃₋ₐ-R'ₐ (S4- II),

worin bedeutet:
- G ist -H, eine Phenylgruppe, -OH, -O-CH₃, -CH₃, -CH₂CH₃, -CH₂CH₂CH₃, -CH (CH₃)₂, -CH₂CH₂CH₂CH₃, -CH₂CH (CH₃)₂, -CH(CH₃)CH₂CH₃, -C(CH₃)₃ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   - -N(R")- CH₂-CH₂- N(R")₂
   - -N(R")₂
   - -N⁺(R")₃A⁻
   - N+H(R")₂A⁻
   - N+H₂(R_{"})A⁻
   - N(R")- CH₂- CH₂- N⁺R"H₂A⁻,
wobei jedes R" für gleiche oder verschiedene Reste aus der Gruppe -H, -Phenyl, -Benzyl, der C₁₋₂₀-alkylreste, vorzugsweise -CH₃, -CH₂CH₃, -CH₂CH₂CH₃, -CH(CH₃)₂, -CH₂CH₂CH₂CH₃, -CH₂CH (CH₃)₂, -CH(CH₃) CH₂CH₃, -C(CH₃)₃, steht und A⁻ ein Anion repräsentiert, welches vorzugsweise ausgewählt ist aus Chlorid, Bromid, Iodid oder Methosulfat.

Besonders bevorzugte aminofunktionelle Silikone entsprechen der Formel (S4-III) 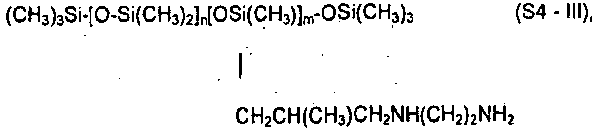 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet.

Besonders bevorzugt sind weiterhin aminofunktionelle Silikone der Formel (S4 - IV) 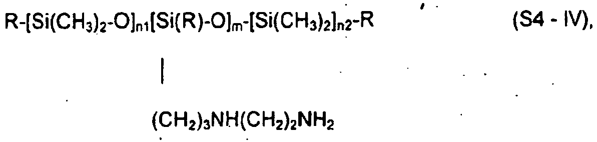 worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone bezeichnet und sind beispielsweise in Form einer Emulsion als Handelsprodukt Dow Corning^{®} 949 im Gemisch mit einem kationischen und eine nichtionischen Tensid erhältlich.

Vorzugsweise werden solche aminofunktionellen Silikone eingesetzt, die eine Aminzahl oberhalb von 0,25 meq/g, vorzugsweise oberhalb von 0,3 meq/g und insbesondere bevorzugt oberhalb von 0,4 meq/g aufweisen. Die Aminzahl steht dabei für die Milli-Äquivalente Amin pro Gramm des aminofunktionellen Silikons. Sie kann durch Titration ermittelt und auch in der Einheit mg KOH/g angegeben werden.

Weitere geeignete Silikone sind beispielsweise
- oligomere Polydimethylcyclosiloxane (INCI-Bezeichnung: Cyclomethicone), insbesondere die tetramere und die pentamere Verbindung, die als Handelsprodukte DC 245 Fluid, DC 344 bzw. DC 345 von Dow Corning vertrieben werden,
- Hexamethyl-Disiloxan (INCI-Bezeichnung: Hexamethyldisiloxane), z. B. das unter der Bezeichnung Abil^{®} K 520 vertriebenen Produkt,
- Polyphenylmethylsiloxane (INCI-Bezeichnung: Phenyl Trimethicone), z. B. das Handelsprodukt DC 556 Cosmetic Grade Fluid von Dow Corning,
- Ester sowie Partialester der Silikon-Glykol-Copolymere, wie sie beispielsweise von der Firma Fanning unter der Handelsbezeichnung Fancorsil^{®} LIM (INCI-Bezeichnung: Dimethicone Copolyol Meadowfoamate) vertrieben werden,
- anionische Silikonöle, wie beispielsweise das Produkt Dow Corning^{®}1784.

In der Regel ist es ausreichend, die Silikonverbindung in einer Menge von 0,01 bis 2 Gew.-%, bezogen auf die Gesamtmenge an kosmetischem Mittel zur temporären Verformung keratinischer Fasern, zuzusetzen. Bevorzugt ist der Einsatz in einer Menge von 0,01 bis 1 Gew.-%, besonders bevorzugt in einer Menge von 0,03 bis 1 Gew.% und ganz besonders bevorzugt in einer Menge von 0,1 bis 1 Gew.%. Es spricht jedoch nichts dagegen, die Silikonverbindung auch in einer größeren Menge zuzugeben, vor allem, wenn durch Einsatz der Silikonverbindung in einem Stylingmittel auch ein pflegender Effekt erzielt werden soll. Als Pflegekomponenten werden Silikonverbindungen oft in Mengen bis zu 25 Gew.-%, eingesetzt.

Die nachfolgenden Beispiele sollen den Gegenstand der vorliegenden Erfindung erläutern ohne ihn in irgendeiner Weise zu beschränken.

### Beispiele

Die im folgenden angegebenen Mengenangaben verstehen sich - soweit nichts anderes vermerkt ist - in Gewichtsprozent.

### 1 Herstellung der Stylingmittel

Es wurden auf übliche Weise die Stylingmittel 1a bis 2b hergestellt, wobei diese folgende Zusammensetzung aufwiesen:

| **Rohstoffe** | **1a** | **1b** | **2a** | **2b** |
|---|---|---|---|---|
| Amphomer^{®} | 9,5 | 9,5 | 8,25 | 8,25 |
| Isopropylmyristat | 0,1 | 0,4 | 0,1 | 0,5 |
| Benzophenone-4 | 0,2 | 0,2 | 0,2 | 0,2 |
| 2-Amino-2-methylpropanol | 1,7 | 1,7 | 1,5 | 1,5 |
| Parfüm | 0,1 | 0,1 | 0,5 | 0,5 |
| Ethanol (96 %), vergällt | 75,0 | 75,0 | 80,0 | 80,0 |
| Wasser, entsalzt | ad 100 | ad 100 | ad 100 | ad 100 |

### 2 Bestimmung der Klebrigkeit

Die Messung der Klebrigkeit erfolgte nach der Stempelmethode nach Kempf, wie sie beispielsweise in Seifen, Öle, Fette, Wachse 131 (2005), Nr. 5, S. 22 und 28 beschrieben ist. Dazu wurde das zu prüfende Stylingmittel mit einem Streichrakelbalken in einer Dicke von 120 µm auf eine Glasplatte aufgetragen und anschließend für eine Stunde unter Raumbedingungen getrocknet. Pro Stylingmittel wurden 3 Glasplatten entsprechend präpariert. Die so mit einem Polymerfilm beschichteten Glasplatten wurden über Nacht in einem Klimaschrank bei 298 K und 90 % relativer Feuchtigkeit gelagert. Mit einem Gummistempel mit einem Durchmesser von 40 mm wurde ein Carbonband (Kunststoff-Carbonband der Firma Fiedler) mit einem Druck von 2 bar für 10 Sekunden auf den Polymerfilm gepresst. Jede beschichtete Glasplatte wurde an vier verschiedenen Stellen entsprechend behandelt. Je klebriger die Polymeroberfläche ist, umso mehr Druckfarbe des Carbonbandes haftet auf dem Polymerfilm.

Zur Auswertung der entstehenden Abdrücke wurden die Glasplatten mit einem Hewlett Packard scanjet 4500c mit Passe-partout gescannt und die so erhaltenen Abbildungen in Schwarz-Weiß-Darstellungen (8 bit, 256 Graustufen) überführt.

Die weitere Auswertung der erhaltenen Schwarz-Weiß-Abbildungen erfolgte mittels der kommerziell erhältlichen Software WinView, mit deren Hilfe sich der Weißanteil einer ausgewählten Fläche der Abbildungen bestimmen lässt. Einer völlig schwarzen Fläche, d.h. einer Fläche ohne Weißanteil wird dabei der Wert 0, einer völlig weißen Fläche der Wert 255 zugeordnet. Da auch die unbedruckten Flächen jeder Glasplatte eine gewisse Färbung, etwa durch den Polymerfilm, aufweisen, wurde zunächst die Weißintensität der unbedruckten Flächen einer Glasplatte bestimmt. Rechnerisch wurde ausgehend von diesem Wert eine Standardisierung auf einen völlig weißen Hintergrund vorgenommen. Anschließend wurde die Weißintensität der 4 bedruckten Flächen jeder Glasplatte bestimmt und ebenfalls standardisiert. Je klebriger der Polymerfilm ist, desto dunkler ist die entstehende Fläche, d.h. desto geringer ist die Weißintensität.

Aus den so bestimmten Werten der Weißintensitäten wird die Klebrigkeit in Form des Grads der Bedeckung der Fläche, die mit dem Carbonband bedruckt wurde, berechnet. Die Werte werden in Prozent angegeben, wobei keine Klebrigkeit einer Bedeckung von 0, d.h. maximaler Weißintensität und maximale Klebrigkeit einer Bedeckung von 100, d.h. minimaler Weißintensität entspricht. Je niedriger die erhaltenen Werte sind, desto geringer ist demnach auch die Klebrigkeit.

Für jedes Stylingmittel werden insgesamt 12 Werte ermittelt (je 3 Glasplatten mit je 4 Abdrücken). Statistische Ausreißer werden mittels Shapiro-Wilks bestimmt und eliminiert und die verbleibenden Werte gemittelt.

Es wurden so folgende Klebrigkeiten ermittelt:

| **Stylingmittel** | 1a | 1b | 2a | 2b |
|---|---|---|---|---|
| **Klebrigkeit** | 21,6 | 17,8 | 24,8 | 21,7 |

Ein Vergleich der Klebrigkeit der Zusammensetzungen 1a und 1b bzw. 2a und 2b zeigt deutlich, dass die Erhöhung des Gehalts an Isopropylmyristat eine deutliche Verringerung der Klebrigkeit bewirkt.

### 3 Verzeichnis der eingesetzten Rohstoffe

Die im Rahmen der Beispiele eingesetzten Rohstoffe sind wie folgt definiert:

| | |
|---|---|
| Amphomer^{®} | INCI-Bezeichnung: Otylacrylamide/Acrylates/Butyl-aminoethyl Methacrylate Copolymer (National Starch) |
| Dow Corning^{®} 190 surfactant | Mit durchschnittlich 18 Mol Propylenoxid und 18 Mol Ethylenoxid alkoxyliertes Polydimethylsiloxan-Derivat (INCI-Bezeichnung: PEG/PPG-18/18 Dimethicone) (Dow Corning) |
| Benzophenone-4 (INCI) | 2-Hydroxy-4-methoxybenzophenon-5-sulfonsäure |

## Patentansprüche

1. Verwendung mindestens eines Fettsäureesters der Formel (I) 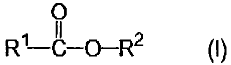 wobei
R¹ für den Kohlenstoffrest einer gesättigten oder ungesättigten, verzweigten oder unverzweigten Fettsäure, und
R² für gegebenenfalls verzweigtes C₁-C₂₀-Alkyl, gegebenenfalls verzweigtes C₁-C₂₀-Monohydroxyalkyl, gegebenenfalls verzweigtes C₂-C₂₀-Dihydroxyalkyl oder gegebenenfalls verzweigtes C₃-C₂₀-Trihydroxyalkyl steht,
zur Reduktion der Klebrigkeit in kosmetischen Mitteln zur temporären Verformung keratinischer Fasern,
wobei die kosmetischen Mittel zur temporären Verformung keratinischer Fasern als filmbildendes und/oder festigendes Polymer mindestens ein Homo- oder Copolymer des Vinylpyrrolidons oder ein amphoteres Copolymer aus Octylacrylamid, Acrylaten und/oder Methacrylaten und Butylaminoethylmethacrylat enthalten.

2. Verwendung gemäß Anspruch 1, **dadurch gekennzeichnet, dass** R¹ für den Kohlenstoffrest einer gesättigten oder ungesättigten, verzweigten oder unverzweigten C₆-C₃₀-Fettsaure steht.

3. Verwendung gemäß wenigstens eines der Ansprüche 1 und 2, **dadurch gekennzeichnet, dass** R¹ für den Kohlenstoffrest einer gesättigten oder ungesättigten, verzweigten oder unverzweigten C₁₂-C₂₀-Fettsäure steht.

4. Verwendung gemäß wenigstens eines der Ansprüche 1 bis 3, **dadurch gekennzeichnet, dass** R¹ für den Kohlenstoffrest einer natürlich vorkommenden Fettsäure steht.

5. Verwendung gemäß wenigstens eines der Ansprüche 1 bis 4, **dadurch gekennzeichnet, dass** R² für gegebenenfalls verzweigtes C₁-C₁₀-Alkyl oder gegebenenfalls verzweigtes C₁-C₁₀-Monohydraxyalkyl steht.

6. Verwendung gemäß wenigstens eines der Ansprüche 1 bis 5, **dadurch gekennzeichnet, dass** als Fettsäureester der Formel (I) Isopropylpalmitat, Isopropylmyristat, 1,2-Butylenglycololeat, 1,3-Butylenglycololeat, 1,4-Butylenglycololeat und/oder 2,3-Butylenglycololeat eingesetzt wird.

7. Verwendung gemäß wenigstens eines der Ansprüche 1 bis 6, **dadurch gekennzeichnet, dass** der mindestens eine Fettsäureester der Formel (I) in einer Menge von 0,1 bis 1 Gew.-%, bezogen auf die Gesamtmenge an kosmetischem Mittel zur temporären Verformung keratinischer Fasern, eingesetzt wird.

8. Verwendung gemäß wenigstens eines der Ansprüche 1 bis 7, **dadurch gekennzeichnet, dass** zusätzlich zu mindestens einem Fettsäureester der Formel (I) eine Silikonverbindung eingesetzt wird.

9. Verwendung gemäß Anspruch 8, **dadurch gekennzeichnet, dass** als Silikonverbindung ein Dimethiconcopolyol eingesetzt wird.

10. Verwendung gemäß wenigstens eines der Ansprüche 8 bis 9, **dadurch gekennzeichnet, dass** die Silikonverbindung in einer Menge von 0,01 bis 1 Gew.-%, bezogen auf die Gesamtmenge an kosmetischem Mittel zur temporären Verformung keratinischer Fasern, eingesetzt wird.

## Claims

1. Use of at least one fatty acid ester of the formula (I) 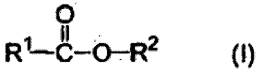 wherein
R¹ denotes the carbon residue of a saturated or unsaturated, branched or unbranched fatty acid, and
R² denotes optionally branched C₁-C₂₀ alkyl, optionally branched C₁-C₂₀ monohydroxyalkyl, optionally branched C₂-C₂₀ dihydroxyalkyl or optionally branched C₃-C₂₀ trihydroxyalkyl,
for reducing tackiness in cosmetic agents for the temporary deformation of keratin fibres,
wherein the cosmetic agents for the temporary deformation of keratin fibres contain as a film-forming and/or setting polymer at least one homopolymer or copolymer of a vinylpyrrolidone or an amphoteric copolymer made from octylacrylamide, acrylates and/or methacrylates and butylamino ethyl methacrylate.

2. Use according to claim 1, **characterised in that** R¹ denotes the carbon residue of a saturated or unsaturated, branched or unbranched C₆-C₃₀ fatty acid.

3. Use according to at least one of claims 1 and 2, **characterised in that** R¹ denotes the carbon residue of a saturated or unsaturated, branched or unbranched C₁₂-C₂₀ fatty acid.

4. Use according to at least one of claims 1 to 3, **characterised in that** R¹ denotes the carbon residue of a naturally occurring fatty acid.

5. Use according to at least one of claims 1 to 4, **characterised in that** R² denotes optionally branched C₁-C₁₀ alkyl or optionally branched C₁-C₁₀ monohydroxyalkyl.

6. Use according to at least one of claims 1 to 5, **characterised in that** the fatty acid ester of the formula (I) which is used is isopropyl palmitate, isopropyl myristate, 1,2-butylene glycol oleate, 1,3-butylene glycol oleate, 1,4-butylene glycol oleate and/or 2,3-butylene glycol oleate.

7. Use according to at least one of claims 1 to 6, **characterised in that** the at least one fatty acid ester of the formula (I) is used in a quantity of 0.1 to 1 % by weight, relative to the total quantity of cosmetic agent for the temporary deformation of keratin fibres.

8. Use according to at least one of claims 1 to 7, **characterised in that** a silicone compound is used in addition to at least one fatty acid ester of the formula (I).

9. Use according to claim 8, **characterised in that** a dimethicone copolyol is used as the silicone compound.

10. Use according to at least one of claims 8 to 9, **characterised in that** the silicone compound is used in a quantity of 0.01 to 1 % by weight, relative to the total quantity of cosmetic agent for the temporary deformation of keratin fibres.

## Revendications

1. Utilisation d'au moins un ester d'acide gras répondant à la formule (I) 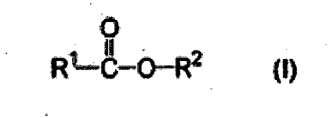 dans laquelle
R¹ représente le résidu d'hydrocarbure d'un acide gras saturé ou insaturé, ramifié ou non ramifié, et
R² représente un groupe alkyle en C₁-C₂₀ facultativement ramifié, un groupe monohydroxyalkyle en C₁-C₂₀ facultativement ramifié, un groupe dihydroxyalkyle en C₂-C₂₀ facultativement ramifié ou un groupe trihydroxyalkyle en C₃-C₂₀ facultativement ramifié,
pour la réduction du caractère collant dans des agents cosmétiques pour la déformation temporaire de fibres kératiniques,
dans laquelle les agents cosmétiques pour la déformation temporaire de fibres kératiniques contiennent comme polymère filmogène ou polymère fixant au moins un homopolymère or copolymère de vinylpyrrolidone ou un copolymère amphotère a base d'octylacrylamide, d'acrylates et/ou de méthacrylates et de méthacrylate de butylaminoethyle.

2. Utilisation selon la revendication 1, **caractérisée en ce que** R¹ représente le résidu d'hydrocarbure d'un acide gras en C₆-C₃₀ saturé ou insaturé, ramifié ou non ramifié.

3. Utilisation selon au moins une des revendications 1 et 2, **caractérisée en ce que** R¹ représente le résidu d'hydrocarbure d'un acide gras en C₁₂-C₂₀ saturé ou insaturé, ramifié ou non ramifié.

4. Utilisation selon au moins une des revendications 1 à 3, **caractérisée en ce que** R¹ représente le résidu d'hydrocarbure d'un acide gras existant dans la nature.

5. Utilisation selon au moins une des revendications 1 à 4, **caractérisée en ce que** R² représente un groupe alkyle en C₁-C₁₀ facultativement ramifié ou un groupe monohydroxyalkyle en C₁-C₁₀ facultativement ramifié.

6. Utilisation selon au moins une des revendications 1 à 5, **caractérisée en ce qu'**on met en oeuvre à titre d'acide gras répondant à la formule (I), le palmitate d'isopropyle, le myristate d'isopropyle, l'oléate de 1,2-butylèneglycol, l'oléate de 1,3-butylèneglycol, l'oléate de 1,4-butylèneglycol et/ou l'oléate de 2,3-butylèneglycol.

7. Utilisation selon au moins une des revendications 1 à 6, **caractérisée en ce qu'**on met en oeuvre au moins un ester d'acide gras répondant à la formule (I) en une quantité de 0,1 à 1% en poids rapporté à la quantité totale d'agents cosmétiques pour la deformation temporaire de fibres kératiniques.

8. Utilisation selon au moins une des revendications 1 à 7, **caractérisée en ce qu'**on met en oeuvre, en plus d'au moins un ester d'acide gras répondant à la formule (I), un composé de silicone.

9. Utilisation selon la revendication 8. **caractérisée en ce qu'**on met en oeuvre à titre de composé de silicone, un copolyol de Dimethicone.

10. Utilisation selon au moins une des revendications 8 à 9, **caractérisée en ce qu'**on met en oeuvre le composé de silicone en une quantité de 0,01 à 1% en poids rapporté à la quantité totale d'agents cosmétiques pour la déformation temporaire de fibres kératiniques.
